Europäisches Patentamt

**European Patent Office**

Office européen des brevets

(11) Publication number: **0 168 136**
**A1**

(12) **EUROPEAN PATENT APPLICATION**

(21) Application number: **85303303.3**

(22) Date of filing: **10.05.85**

(51) Int. Cl.⁴: **A 61 M 1/30**
**A 61 M 25/00**

(30) Priority: **15.05.84 US 610542**

(43) Date of publication of application:
**15.01.86 Bulletin 86/3**

(84) Designated Contracting States:
**BE CH DE FR GB IT LI LU NL**

(71) Applicant: **SHILEY INCORPORATED**
**17600 Gilette**
**Irvine California(US)**

(72) Inventor: **Edelman, William**
**4873 Dogwood Avenue**
**Seal Beach California(US)**

(74) Representative: **Moore, James William et al,**
**Pfizer Limited Ramsgate Road**
**Sandwich Kent CT13 9NJ(GB)**

(54) **Dual lumen subclavian cannula.**

(57) A dual lumen subclavian cannula for use in hemodialysis comprises a tube (1) divided into lumens (7,8) by a longitudinal septum (6). The distal end of the tube (1) is tapered to a blunt tip (15) terminating in an aperture (17) communicating with the blood inlet lumen (7). The blood return lumen (8) has an aperture (21) in the tube wall, and is closed beyond a point proximal to the tapered section (15) of the tube by a biocompatible inert material (23).

Fig.1.

0168136

D.P.C. 6807

## DUAL LUMEN SUBCLAVIAN CANNULA

This invention relates to a dual lumen subclavian cannula for medical use, and more particularly to semi-permanent means for simultaneously withdrawing blood from the subclavian vein for extracorporeal processing and returning the treated blood back to the vein.

Dual lumen cannulae have been proposed and used for hemodialysis because they provide the advantage of simultaneously withdrawing and returning blood of a patient through a single puncture rather than two. Such cannulae have been made of rigid metal tubes and are in general of two types: those with concentric flow lumens such as disclosed in U.S. Patent No. 4,037,599 and those with parallel channels as in U.S. Patent No. 4,203,436. These types are only suitable for a single blood treatment and must be emplaced in and removed from the patient for each use. None of them is flexible or suitable for emplacement in the subclavian vein. While these cannulae have proven useful, there would be an advantage for a cannula that can be left in place for several uses.

Single lumen cannulae suitable for semipermanent placement in the subclavian vein are known. These can be left in place for one to two weeks. One such type is the "Vas-Cath" subclavian cannula sold in the United States by Shiley Incorporated. These devices perform satisfactorily, but they require a more complex hemodialysis apparatus that must sequentially take in blood, treat it, and return the treated blood to the patient. These steps cannot be done simultaneously as is most desirable.

U.S. Patent No. 4,403,983 discloses a dual lumen cannula with a blood return lumen terminating in a tapered, closed blunt point. The distal end of the tube wall enclosing the blood inlet lumen is cut off to create an opening opposite the proximal end of the tapered section of the blood return lumen.

The present invention relates to a dual lumen subclavian cannula suitable for semi-permanent use comprising a flexible tube divided into first and second flow lumens by a flexible longitudinal septum. The flexible tube is preferably of circular cross section and the septum is preferably positioned on a diameter of said cross section. The septum is substantially centrally located within the tube and extends from the proximal end to the distal tip of the tube. The proximal end of the tube is fitted with a manifold for separately collecting fluid from the first lumen and distributing fluid into the second lumen. The distal end of the tube is tapered to a blunt tip and terminates in an aperture communicating with the second lumen. The second flow lumen, preferably, has at least one aperture in the tube wall proximal to the opening at the distal tip. The distal opening for the first lumen is provided by at least one aperture in the tube wall proximal to the tapered section of the tube. The first lumen is closed beyond a point proximal to the tapered section of the tube by a biocompatible inert material, preferably radiopaque fluorinated ethylene-propylene copolymer, filling the space between the septum and the flexible tube.

It is the primary object of this invention to provide a dual lumen cannula, useful for hemodialysis, that can be implanted in a blood vessel of a patient and left in place for more than one treatment.

It is another object to provide a dual lumen cannula where distal inlet and return openings are sufficiently separated to avoid mixing of treated and untreated blood flows.

It is another object to provide a dual lumen cannula in which blood clotting is inhibited.

It is still another object of this invention to provide a dual lumen cannula that provides an improvement resulting in increased blood flow over the cannula described in United States Patent 4,403,983.

FIG. 1 is a longitudinal section view of one embodiment of the invention.

FIG. 2 shows a cross section of the cannula tube taken along line 2-2 in FIG. 1.

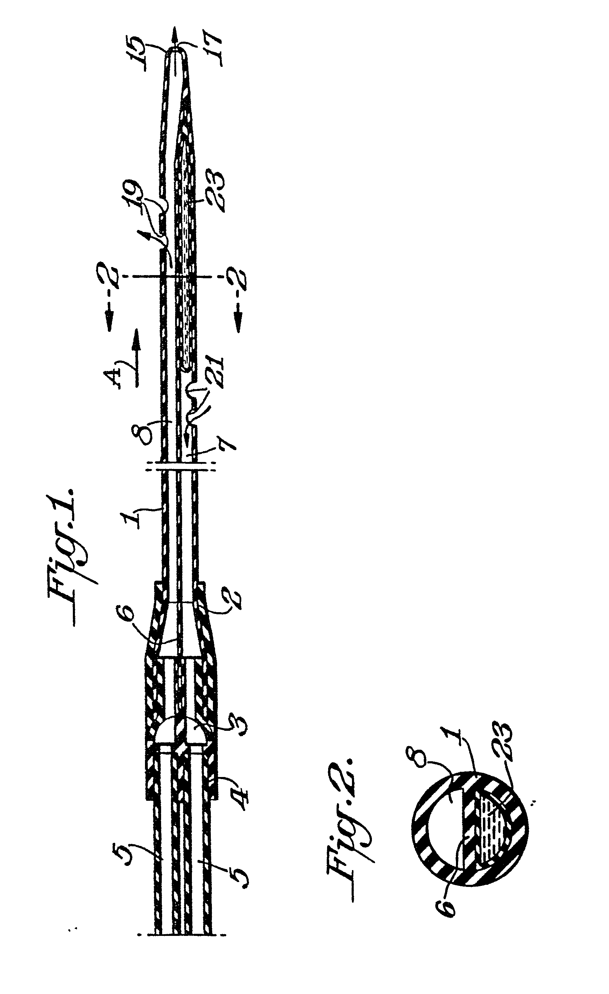

The components of a cannula according to this invention can be seen in FIG. 1. It comprises the cannula tube 1, divided longitudinally by septum 6 to create two parallel lumens 7 and 8. Tube 1 is attached to a flow manifold assembly by locking ring 2. The manifold comprises head unit 3 sealed to base 4. Locking ring 2 joins the cannula tube 1 securely to head 3, as by ultrasonic sealing. Conduits 5 are jointed to base 4 by insertion into female fittings in base 4 to form liquid tight seals. These conduits carry blood in separate streams simultaneously back and forth between the manifold assembly and the extracorporeal blood processing unit, not shown. Arrow A indicates the direction of blood flow surrounding the cannula tube when it is properly emplaced in a vein. The proximal end of septum 6 is also sealed into head 3 to maintain separation of blood flow from the cannula tube lumens into the manifold. The first lumen 7 is the blood inlet lumen and the second lumen 8 is the blood return lumen. A cross section of tube 1 is shown as FIG. 2.

The tube 1 tapers to a distal blunt tip 15. The blunt tip 15 is provided with an opening 17 which communicates with the second lumen 8. A second opening in communication with lumen 8 is created by an aperture 19 in the tube wall proximal to the opening 17 at the distal blunt tip 15.

The first lumen 7 is provided with a distal opening created by at least one aperture 21 in the tube wall. The aperture 21 is located proximal to the tapered section of the tube. The first lumen 7 is closed beyond a point proximal to said tapered section. The closed portion is filled with a biocompatible material 23. This material substantially fills the space between the septum 6 and the flexible tube 1. The material 23 is preferably radiopaque, most preferably radiopaque fluorinated ethylenepropylene copolymer.

The blood carrying conduits 5 are composed of flexible blood compatible plastics such as silicone rubber or flexible vinyl tubing and usually lead to Luer fittings for convenient and secure connection to the blood treatment apparatus. The Luer fittings can be capped off when the cannula is not in use. These conduits are made flexible for convenience in closing.

Because the cannula tube will be semipermanently emplaced in a patient's blood vessel, the tube must be made of material such as ethylene-propylene copolymer suitable for long exposure within the human body. The tube is preferably made in one piece. It must have a relatively smooth surface and have a significantly smaller outside diameter than the inside diameter of the blood vessel selected for use. It must be flexible enough to follow and adapt to the natural curvature of the blood vessel and yet not kink on bending to constrict blood flow. The tube dimensions should vary depending on patient size and the blood vessel used. However, in a preferred embodiment, suitable for the average adult subclavian vein, the tube and septum are made of flexible fluorinated ethylene-propylene copolymer (FEP) because of its good biocompatibility and to facilitate precise location of the distal cannula tip by X-ray observation. This preferred cannula tube is of circular cross section and has the following dimensions: length about 200 mm; outside diameter about 4 mm; wall thickness about 0.6 mm; septum thickness about 0.6 mm. The distal holes creating openings in each lumen are preferably 2 mm. diameter holes spaced about 1 mm apart.

The manifold parts and locking ring are preferably made of transparent polycarbonate plastic. The manifold is preferably made transparent to allow visual observation of blood flow.

The design of this cannula minimizes blood clotting within the device during use by avoiding stagnant flow zones. In the prior art concentric tube dual lumen cannulae, the inner lumen tube unavoidably comes in contact with the outer tube wall, especially when the cannula is bent. This creates zones, where the tube surfaces meet at acute angles, of relatively slow flowing blood. These stagnant areas promote clotting of the blood with attendant danger to the patient. The cannula of this invention avoids this problem because the cross section of the lumens contains no acute angles causing stagnant zones. Further, the cross section does not significantly change on bending.

The major flow of blood out of the second lumen is through the opening of the distal tip. The blood return is through the aperture or apertures in the tube wall in the first lumen. There is complete segregation of the treated and non-treated blood. When the cannula is properly emplaced in the vein, the cannula tip is pointing in the direction of the blood flow as shown by arrow A in Fig. 1. Consequently, there is no recirculation of treated blood back into the inlet lumen.

The cannula is preferably emplaced with the help of a guide wire by the Seldinger technique. The guide wire is threaded through the second lumen and exits at the open distal tip. In this way the open distal tip facilitates the implantation of the catheter. The open distal tip permits the guide wire to pass freely through the cannula as the cannula slides into position over the wire. The distal tip is tapered to a blunt point to facilitate the entrance of the cannula into the body. The cannula, being flexible, will accomodate itself to any slight curvature in the vein. If desired, the cannula tube can be prebent by hand into an expected curve.

Once in place, this cannula can be left in the vein for several weeks. During this period, the patient does not have to be hospitalized. He can live a reasonably normal life with the cannula left in place between the two to three periods of active hemodialysis a week. An obvious advantage of this semi-permanent emplacement is that many fewer vein punctures are necessary, avoiding extensive scarring. Thus, useful vein sites for possible future and emergency use are preserved.

This invention has been described by reference to preferred embodiments but is not limited to such embodiments.

D.P.C. 6807

## CLAIMS

1.   A dual lumen subclavian cannula suitable for semi-permanent use, comprising;

a flexible tube (1) divided into first (7) and second (8) flow lumens by a flexible longitudinal septum (6) substantially centrally located within said tube and extending from the proximal end to the distal tip of said tube;

the proximal end of said tube (1) being fitted to a manifold for separately collecting fluid from said first lumen (7) and distributing fluid into said second lumen (8); characterized in that

the distal end of said tube is tapered to a blunt tip (15), terminating in an aperture (17) communicating with said second lumen (8);

a distal opening for said first lumen (7) being provided by at least one aperture (21) in the tube wall proximal to the tapered section of said tube (1); and

said first lumen (7) being closed beyond a point proximal to the tapered section of said tube (1) by a biocompatible inert material (23) filling the space between said septum (6) and said flexible tube (1).

2.   A cannula according to claim 1 characterized in that said tube (1), said septum (6), and said inert filling material (23) are radiopaque fluorinated ethylenepropylene copolymer.

3.   A cannula according to claim 1 characterized in that the second flow lumen (8) has at least one aperture in the tube wall proximal to the opening at the distal tip.

4.   A cannula according to claim 1 characterized in that said tube is of circular cross section and said septum (6) is positioned on a diameter of said circular cross section.

*Fig.1.*

*Fig.2.*

## European Patent Office

## EUROPEAN SEARCH REPORT

Application number

| | DOCUMENTS CONSIDERED TO BE RELEVANT | | EP 85303303.3 |
|---|---|---|---|

| Category | Citation of document with indication, where appropriate, of relevant passages | Relevant to claim | CLASSIFICATION OF THE APPLICATION (Int Cl 4) |
|---|---|---|---|
| D,A | US - A - 4 403 983 (W. EDELMAN et al.)<br>* Totality * | 1-4 | A 61 M 1/30<br>A 61 M 25/00 |
| A | EP - A1 - 0 101 890 (K. AIGNER)<br>* Totality; especially fig. 2, 2a,3a; page 13, line 18 - page 14, line 15 * | 1,3,4 | |
| A | US - A - 4 385 631 (U. UTHMANN)<br>* Totality * | 1,3 | |
| A | US - A - 4 098 275 (D.V. CONSALVO)<br>* Totality * | 1,4 | |
| | | | TECHNICAL FIELDS SEARCHED (Int Cl 4)<br><br>A 61 M 1/00<br>A 61 M |

The present search report has been drawn up for all claims

| Place of search | Date of completion of the search | Examiner |
|---|---|---|
| VIENNA | 01-10-1985 | LUDWIG |